(19) Europäisches Patentamt
European Patent Office
Office européen des brevets

(11) Veröffentlichungsnummer: **0 410 554 A2**

(12) # EUROPÄISCHE PATENTANMELDUNG

(21) Anmeldenummer: **90250192.3**

(22) Anmeldetag: **27.07.90**

(51) Int. Cl.[5]: **C07J 53/00**, A61K 31/565

(30) Priorität: **28.07.89 DE 3925507**

(43) Veröffentlichungstag der Anmeldung:
**30.01.91 Patentblatt 91/05**

(84) Benannte Vertragsstaaten:
**AT BE CH DE DK ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Bohlmann, Rolf, Dr.**
**Kühler Weg 6a**
**D-1000 Berlin 19(DE)**
Erfinder: **Künzer, Hermann, Dr.**
**Kösliner Strasse 19**
**D-1000 Berlin 65(DE)**
Erfinder: **Wiechert, Rudolf, Prof. Dr.**
**Petzower Strasse 8a**
**D-1000 Berlin 39(DE)**
Erfinder: **Henderson, David, Dr.**
**Jahnstrasse 17**
**D-1000 Berlin 28(DE)**
Erfinder: **Schneider, Martin Dr.**
**Fasanenstrasse 62**
**D-1000 Berlin 12(DE)**
Erfinder: **Nishino, Yukishige, Dr.**
**Lanzendorfer Weg 14**
**D-1000 Berlin 22(DE)**

(54) **14,17 alfa-Ethano- und Ethanoestratriene, Verfahren zur Herstellung dieser Verbindungen, sowie ihre Verwendung zur Herstellung von Arzneimitteln.**

(57) Es werden neue 14,17α-Etheno- und Ethanoestratriene der allgemeinen Formel I

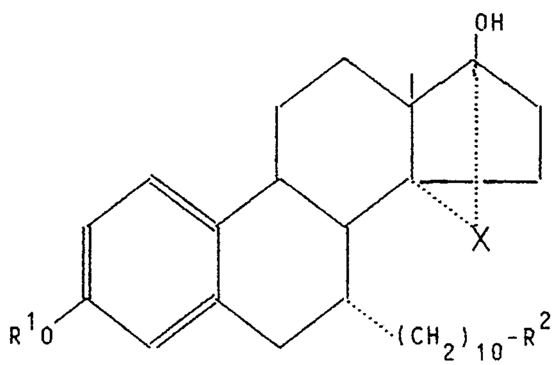

(I),

beschrieben, worin
X für eine $-CH_2-CH_2-$ oder

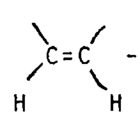

Brücke,

EP 0 410 554 A2

$R^1$ für ein Wasserstoffatom, eine $C_1$- bis $C_{15}$-Acyl-, Benzoyl-, $C_1$- bis $C_{15}$-Alkyl-, $C_3$- bis $C_9$-Cycloalkyl- oder Alkylcycloalkylgruppe und
$R^2$ für die Gruppierung

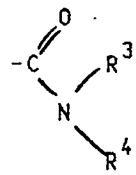 oder 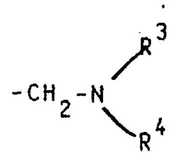 ,

wobei $R^3$ und $R^4$ unabhängig voneinander ein Wasserstoffatom oder eine geradkettige oder verzweigte, gegebenenfalls teilweise oder vollständig fluorierte $C_1$-$C_8$-Alkylgruppe bedeuten,
stehen,
ein Verfahren zu deren Herstellung, diese Verbindungen enthaltende pharmazeutische Präparate, sowie ihre Verwendung zur Herstellung von Arzneimitteln.
Die neuen Verbindungen besitzen starke antiestrogene Wirksamkeit.

# 14,17α-ETHENO- UND -ETHANOESTRATRIENE, VERFAHREN ZUR HERSTELLUNG DIESER VERBINDUNGEN, SOWIE IHRE VERWENDUNG ZUR HERSTELLUNG VON ARZNEIMITTELN.

Die vorliegende Erfindung betrifft 14,17α-Etheno- und -Ethanoestratriene der allgemeinen Formel I

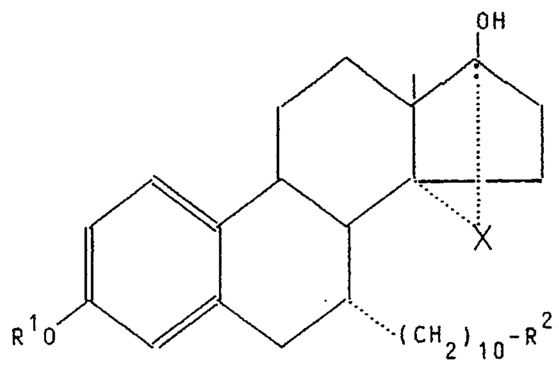

(I),

worin
X für eine $-CH_2-CH_2-$ oder

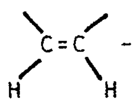

Brücke,
$R^1$ für ein Wasserstoffatom, eine $C_1$- bis $C_{15}$-Acyl-, Benzoyl-, $C_1$- bis $C_{15}$-Alkyl-, $C_3$- bis $C_9$-Cycloalkyl- oder Alkylcycloalkylgruppe und
$R^2$ für die Gruppierung

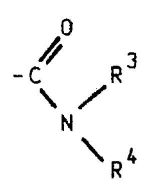

oder

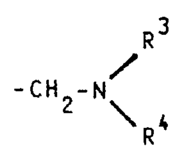

,

wobei $R^3$ und $R^4$ unabhängig voneinander ein Wasserstoffatom oder eine geradkettige oder verzweigte gegebenenfalls teilweise oder vollständig fluorierte $C_1$-$C_8$-Alkylgruppe bedeuten,
stehen,
ein Verfahren zu deren Herstellung, diese Verbindungen enthaltende pharmazeutische Präparate, sowie ihre Verwendung zur Herstellung von Arzneimitteln.

In den Verbindungen dieser Erfindung werden als $C_1$-$C_{15}$ Acylgruppen Formyl, Acetyl, i-Propanoyl, n-Propanoyl, und die Isomeren von Butanoyl, Pentanoyl, Heptanoyl, Decanoyl, Dodecanoyl usw. bevorzugt. Geeignete Alkylgruppen sind zum Beispiel Methyl, Ethyl, i-Propyl, n-Propyl, i-, n-, sec.-, t-Butyl, die Isomeren von Penyl, Hexyl, Octyl und, für die Alkylgruppe $R^1$, die Isomeren von Decyl, Undecyl, Dodecyl usw.. Als $R^3$ und $R^4$ sind die teilweise oder vollständig fluorierten Alkyle entsprechend den oben genannten $C_1$-$C_8$-Alkylgruppen geeignet. Der Grad der Fluorierung kann von Monofluorierung bis zur vollständigen Fluorierung reichen.

Geeignete Cycloalkyl- und Alkylcycloalkylgruppen können jeweils 3 bis 9 Kohlenstoffatome enthalten. Die Cycloalkylgruppen in den Alkylcycloalkylgruppen können zum Beispiel Cyclopropyl, Cyclobutyl, Cyclopentyl, Cyclohexyl, Cycloheptyl, Cyclooctyl oder Cyclononyl bedeuten, während die Alkyle den bereits oben für $R^1$, $R^3$ und $R^4$ genannten Alkylgruppen entsprechen. Alle Alkyle bedeuten sowohl gerad- wie auch verzweigtkettige Gruppen.

Bevorzugte Ausführungsformen der vorliegenden Erfindung ergeben sich aus den Merkmalen der Unteransprüche.
Steroidale Antiestrogene, die im wesentlichen frei von einer estrogenen Restwirkung sind (reine Antiestrogene), gehen aus der EP-A 0 138 504 hervor. Aus der Vielzahl der dort beschriebenen Substanzen ist insbesondere das 11-(3,17β-Dihydroxy-1,3,5(10)-estratrien-7α-yl)-undecansäure-(N-butyl-N-methyl)-amid hervorzuheben.
Die besonders hohe orale Wirksamkeit entsprechender, am amidischen Stickstoffatom eine 2,3,4-Heptafluorbutylgruppe tragende, steroidaler Antiestrogene wird von J. Bowler et al. in Steroids 89, S. 71 beschrieben.

Eine Erhöhung der estrogenen Wirksamkeit gegenüber Estradiol bei peroraler Applikation durch Einführung einer 14α, 17α-Etheno- oder Ethanoüberbrückung wurde bei den in der internationalen Patentanmeldung WO 88/01275 beschriebenen 14,17α-Etheno- und Ethanoestratrienen beobachtet, die am C-7-Atom zwei Wasserstoffatome als Substituenten tragen.

Es wurde nunmehr gefunden, daß die erfindungsgemäßen Verbindungen der allgemeinen Formel I bei peroraler Applikation reine Antiestrogene mit stärkerer antiestrogener Wirkung als die in der EP-A 0 138 504 beschriebenen Substanzen sind.

Zur Feststellung der antiestrogenen Wirkung der erfindungsgemäßen Verbindungen wurde die Hemmung des estradiol-induzierten Uterus- und Vaginawachstums an der adulten, ovariektomierten Ratte nach peroraler Verabreichung stellvertretend von 11-(3,17-Dihydroxy-14α,17α-ethano-1,3,5(10)-estratrien-7α-yl)-undecansäure(N-butyl-N-methyl)-amid ( A ) bestimmt. Als Vergleichssubstanz diente 11-(3,17β-Dihydroxy-1,3,5(10)-estratrien-7α-yl)-undecansäure-(N-butyl-N-methyl)-amid ( B ).

Ermittlung der antiestrogenen Wirkstärke nach peroraler Applikation:

Geschlechtsreife, weibliche Ratten (Gewicht ca. 200 g, 6 Tiere/Dosis) werden 14 Tage vor Behandlungsbeginn ovariektomiert. Die Substanzen, gelöst in Benzylbenzoat/Rizinusöl (1+9), werden peroral an 5 aufeinanderfolgenden Tagen appliziert. Gleichzeitig erhalten die Tiere 0,3μg Estradiol im selben Vehikel s.c. verabreicht. Am Tag nach der letzten Applikation werden die Tiere getötet, Uteri und Vaginae herauspräpariert und das Feuchtgewicht bestimmt.
Die Organgewichte werden auf 100 g Körpergewicht bezogen. Die Mittelwerte und Standardabweichungen werden errechnet. Zur Ermittlung der antiestrogenen Wirkstärke wird die prozentuale Hemmung des Organwachstums bezogen auf den Estradiol-behandelten Standard berechnet.
A verursacht eine dosisabhängige und ausgeprägte Hemmung des Uterus- und VaginaWachstums. Bei einer Dosis von 30 mg/kg wird eine nahezu vollständige Hemmung auf das Niveau der ovariektomierten Kontrolle erreicht.
Der antiestrogene Effekt von A ist sowohl bei der maximalen Hemmwirkung wie auch hinsichtlich der Dosis der Wirkung der als Standard anzusehenden Verbindung B bei dieser peroralen Applikation überlegen. Diese Unterschiede sind beim Uterusgewicht in einer Dosis von 3 und 30 mg/kg, beim Vaginagewicht in der 30 mg/kg-Dosis statistisch absicherbar ($p < 0.05$).

Diese Ergebnisse sind in der Tabelle 1 zusammengefaßt und für die Uterigewichte in Abb. 1 sowie für die Vaginaegewichte in Abb. 2 einander vergleichend gegenübergestellt.
Eine estrogene Restwirkung von A nach subcutaner Verabreichung ist weder an der infantilen Maus noch an der infantilen Ratte nachzuweisen, wie aus dem nachfolgend beschriebenen Test hervorgeht; dieser zeigt außerdem, daß die erfindungsgemäße Verbindung A auch s.c. verabreicht ein stark wirksames Antiestrogen bei der infantilen Ratte ist.

Bestimmung der estrogenen/antiestrogenen Wirkung an der infantilen Maus/Ratte.

Weibliche infantile Mäuse (21 Tage alt, Gewicht 13-15 g, 6 Tiere/Dosis) erhalten die Testsubstanz s.c. gelöst in Benzylbenzoat/Rizinusöl (1+9) an 3 (Maus) bzw. 5 (Ratte) aufeinanderfolgenden Tagen entweder allein (Estrogen-Test) oder zusamnmen mit Estradiol (0,1 μg/Maus; 0,3 ug/Ratte), (Antiestrogentest). Am Tag nach der letzten Applikation werden die Tiere getötet, Uteri und Vaginae herauspräpariert und das Feuchtgewicht bestimmt.

Die Organgewichte werden auf 10 g (Maus) bzw. 100 g (Ratte) Körpergewicht bezogen. Die Mittelwerte und Standardabweichungen werden errechnet. Zur Ermittlung der antiestrogenen Wirkstärke wird die prozentuale Hemmung des Organwachstums bezogen auf den Estradiol-behandelten Standard berechnet.

Die Ergebnisse bei infantilen Ratten sind in Tabelle 2 aufgeführt; Abbildung 3 zeigt, daß bei der infantilen Maus die Verbindung A und die Vergleichssubsstanz B bei einer Dosis von 30 mg/kg s.c. keine signifikante estrogene Wirkung an der Vagina und am Uterus aufweisen (Abbildung 4).

Die erfindungsgemäßen Verbindungen eignen sich somit zur Therapie von östrogenabhängigen Erkrankungen, zum Beispiel anovulatorischer Infertilität, Prostatahyperplasie, Mammacarcinom, Endometriumcarcinom und Melanom.

Die tägliche Dosis zur Behandlung der genannten Krankheiten beträgt typischerweise 0,1 bis 25 mg/kg; beim Menschen entspricht dies einer täglichen Dosis von 5 bis 1250 mg. Eine Dosiseinheit enthält erfindungsgemäß 5 bis 500 mg einer oder mehrerer Verbindungen der allgemeinen Formel I.

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H.P. Fiedler, Lexikon der Hilfsstoffe für Pharmazie, Kosmetik und angrenzende Gebiete Cantor KG. Aulendorf in Württemberg 1971.

Die Verbindungen können oral oder parenteral, beispielsweise intraperitoneal, intramuskulär, subkutan oder perkutan, verabreicht werden. Die Verbindungen können auch in das Gewebe implantiert werden. Die zu verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,01-100 mg/kg Körpergewicht, vorzugsweise 0,1-25 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

TABELLE 1

| ANTIÖSTROGENE WIRKUNG AN DER ADULTEN, OVARIEKTOMIERTEN RATTE | | | | |
|---|---|---|---|---|
| Verbindung Dosis/Tag | Uterusgewicht (mg/100 g) | % Hemmung | Vaginagewicht (mg/100 g) | % Hemmung |
| Kontrolle | 44± 3 | - - | 23±7 | - - |
| Estradiol (E2) 0.3 µg/Tier | 187±41 | - - | 55±3 | - - |
| Verbindung A 3 mg/kg | 111±24* | 53 | 51±6 | 13 |
| Verbindung A 30 mg/kg | 56± 8* | 92 | 25±6* | 92 |
| Verbindung B 3 mg/kg | 148±23 | 27 | 46±5* | 29 |
| Verbindung B 30 mg/kg | 83±15* | 73 | 41±6* | 44 |

* = signifikant ($p < 0.05$).

TABELLE 2

| EINFLUSS VON VERBINDUNG A UND B AUF DAS ESTRADIOL-STIMULIERTE UTERUS- UND VAGINAWACHSTUM BEI INFANTILEN RATTEN | | | | |
|---|---|---|---|---|
| Verbindung Dosis/Tag | Uterusgewicht (mg/100 g) | % Hemmung | Vaginagewicht (mg/100 g) | % Hemmung |
| Kontrolle | 68,0±15,3* | | 37,5± 4,9* | |
| Estradiol (E2) 0,3 µg/Tier | 248,6±35,9 | | 82,4± 9,6 | |
| Antiestrogentest Verbindung A 30 mg/kg + E2 | 109,6±10,9* | 77 | 47,9± 7,6* | 77 |
| Estrogentest Verbindung A 30 mg/kg | 49,7±11,1* | | 27,5± 5,9* | |

* = signifikant ($p < 0.05$).

Die Verbindungen der allgemeinen Formel I werden erfindungsgemäß hergestellt, indem in der Verbindung der allgemeinen Formel II

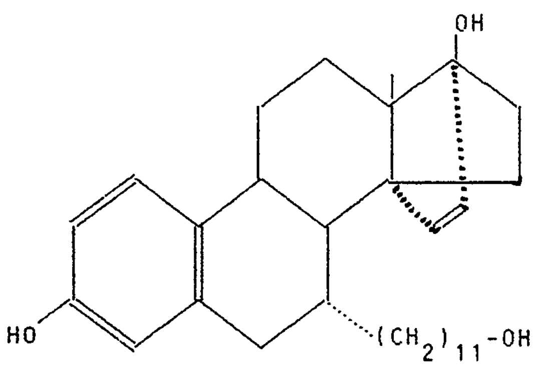

(II)

die Doppelbindung der 14α, 17α-Ethenobrücke gegebenenfalls hydriert, die 3-Hydroxyfunktion geschützt, die 11-Hydroxygruppe der 7α-Undecylseitenkette zur Carbonsäure oxidiert, mit dem Amin $H\text{-}NR^3R^4$, worin $R^3$ und $R^4$ die in Formel I bei der Definition der Gruppierung $R^2$ angegebene Bedeutung haben, aus der Carbonsäure das entsprechende Carbonsäureamid hergestellt, die Schutzgruppe in 3-Stellung gegebenenfalls abgespalten und anschließend gegebenenfalls die 3-Hydroxygruppe entweder partiell acetyliert oder methyliert und/-oder gegebenenfalls die Ketogruppe des Carbonsäureamids vollständig reduziert wird.

Das nachfolgende Beispiel dient zur Erläuterung der Erfindung.

Durch Verwendung eines in den Alkylketten $R^3$ und/oder $R^4$ entsprechend fluorierten Amins im Verfahrensschritt p) lassen sich analog zu dem beschriebenen Schritt p) die seitenkettenfluorierten erfindungsgemäßen Verbindungen herstellen. Die seitenkettenfluorierten Amine sind käuflich oder sind nach dem Fachmann vertrauten Methoden zugänglich.

Die partielle Acylierung oder Alkylierung dei 3-Hydroxygruppe erfolgt nach gängigen Methoden. Eventuell ist ein Schutz der 17-Hydroxygruppe erforderlich.

Die vollständige Reduktion der Ketogruppe des Carbonsäureamids kann mit Lithiumaluminiumhydrid oder ähnlichen Reduktionsmitteln nach ebenfalls üblichen Verfahren erfolgen.

Abb. 1

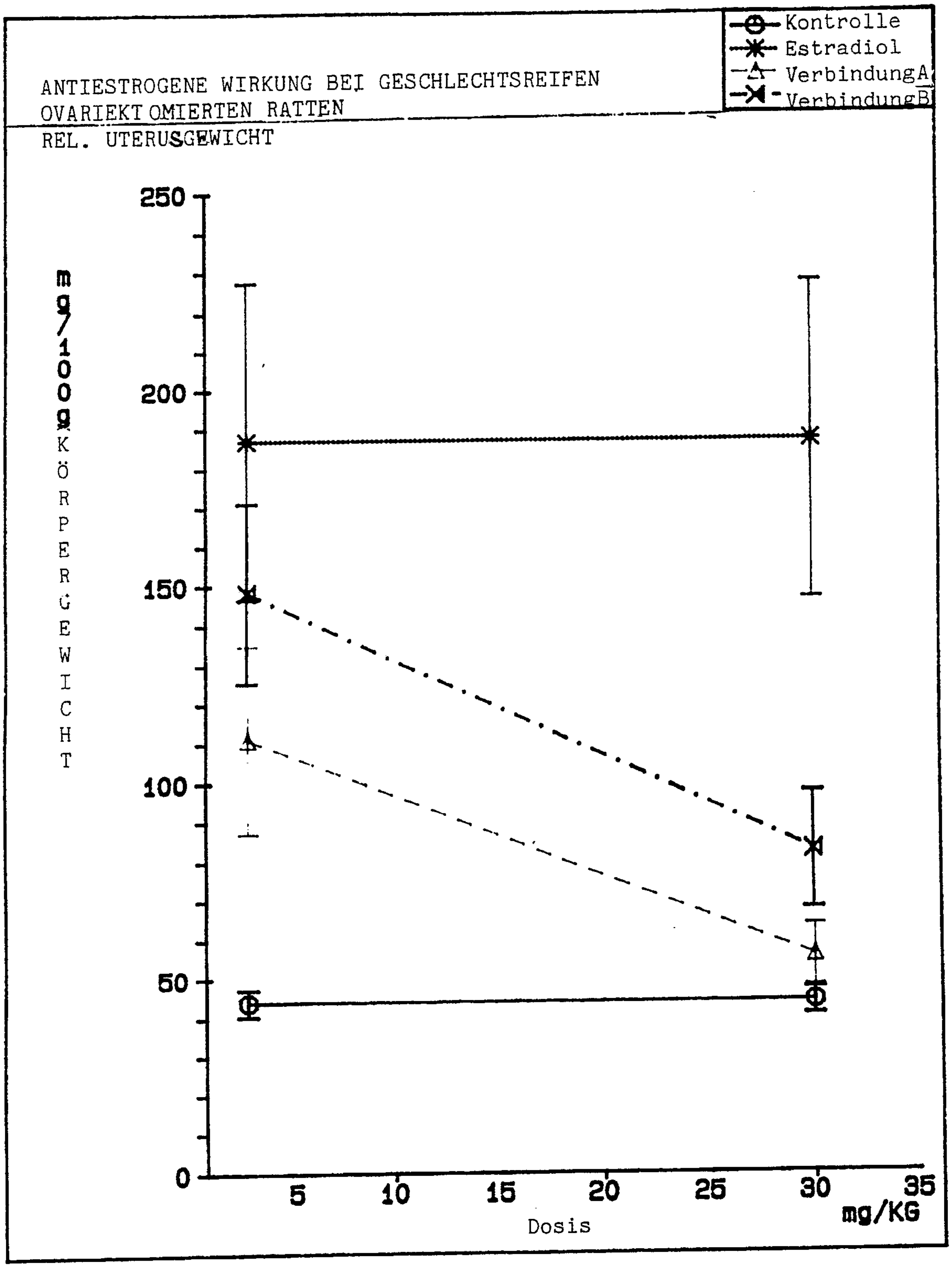
Kontrolle
Estradiol
VerbindungA
VerbindungB
ANTIESTROGENE WIRKUNG BEI GESCHLECHTSREIFEN
OVARIEKTOMIERTEN RATTEN
REL. UTERUSGEWICHT
mg/100g KÖRPERGEWICHT
250
200
150
100
50
0
5
10
15
20
25
30
35
mg/KG
Dosis

Abb. 2

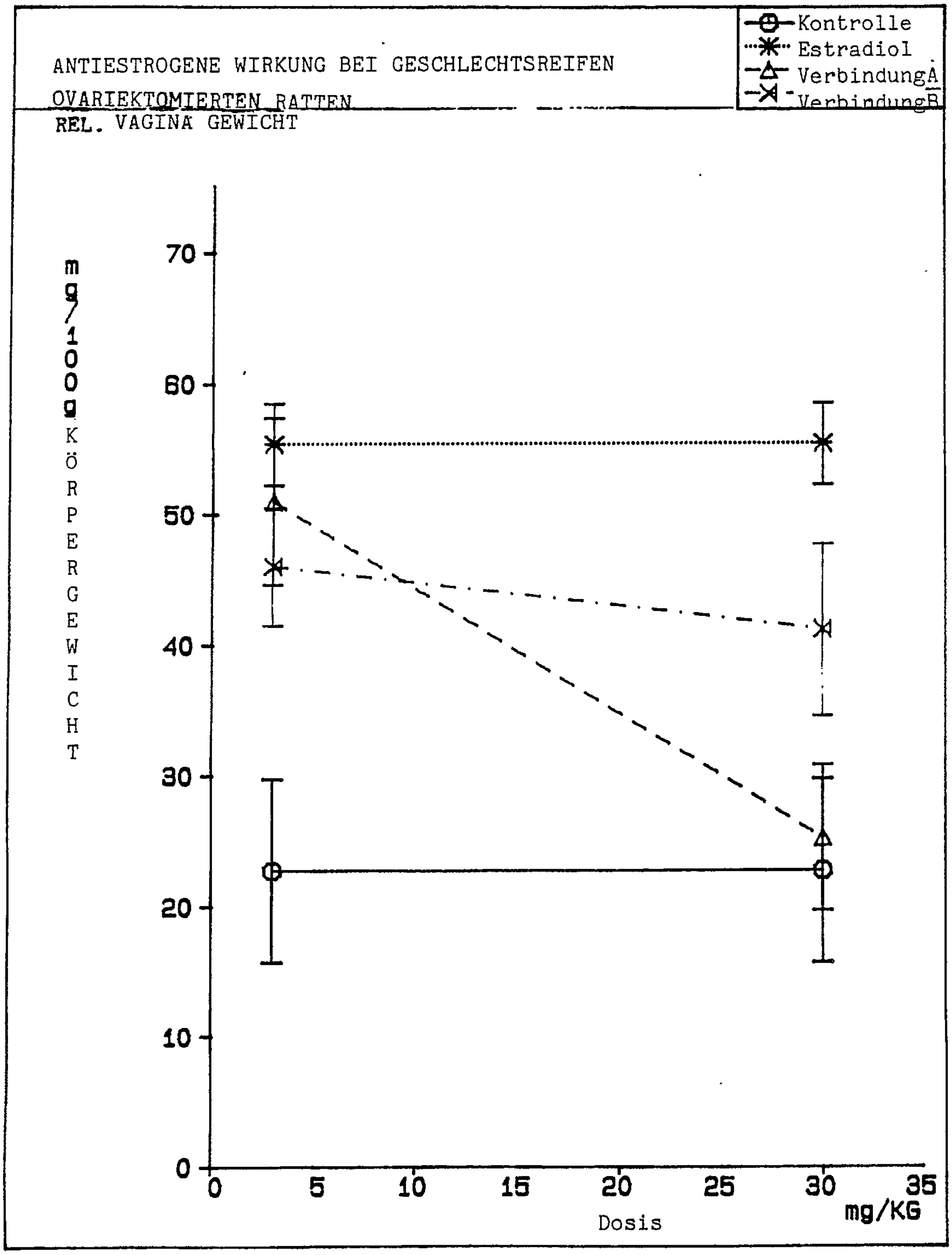
ANTIESTROGENE WIRKUNG BEI GESCHLECHTSREIFEN OVARIEKTOMIERTEN RATTEN
REL. VAGINA GEWICHT
Kontrolle
Estradiol
VerbindungA
VerbindungB
mg/100g KÖRPERGEWICHT
70
60
50
40
30
20
10
0
0
5
10
15
20
25
30
35
mg/KG
Dosis

Abb. 3

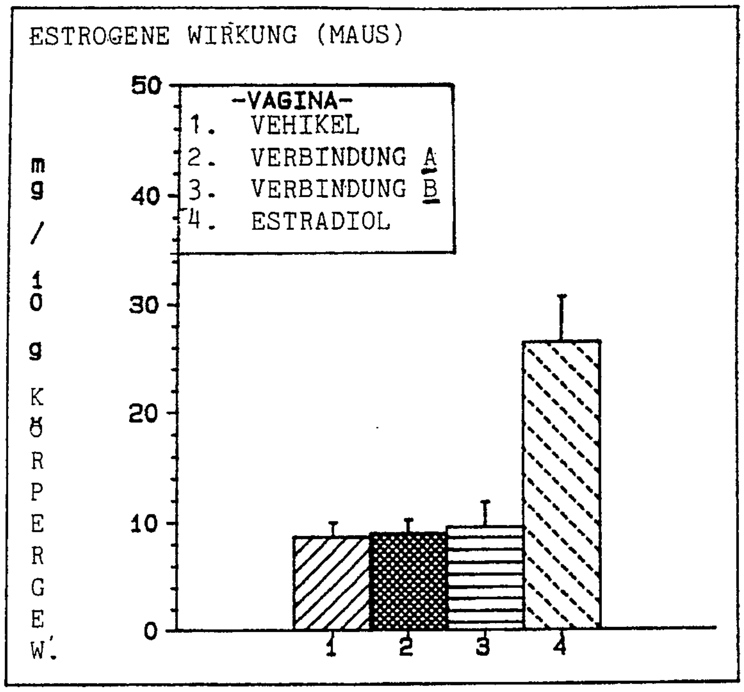
ESTROGENE WIRKUNG (MAUS)
-VAGINA-
1. VEHIKEL
2. VERBINDUNG A
3. VERBINDUNG B
4. ESTRADIOL
mg / 10 g KÖRPERGEW.
50
40
30
20
10
0
1
2
3
4

Abb. 4

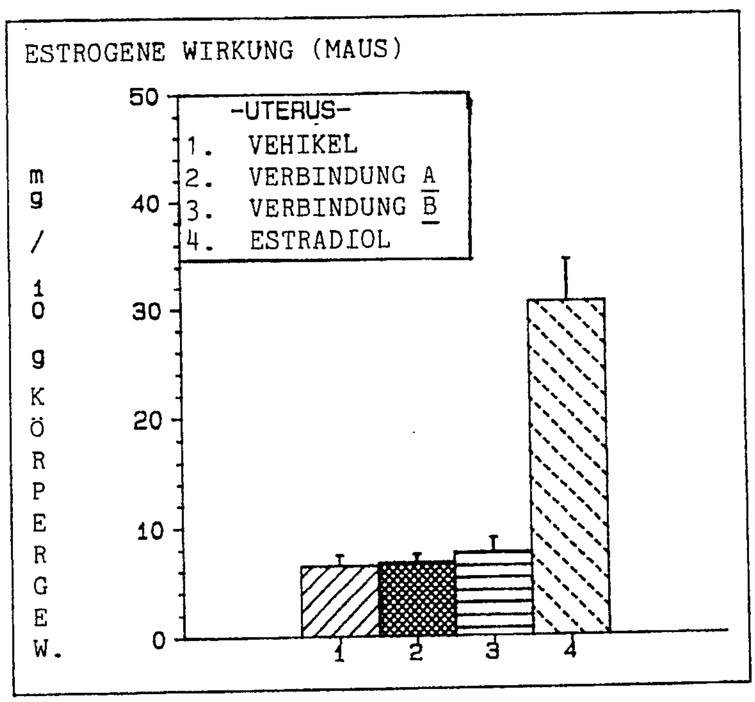
ESTROGENE WIRKUNG (MAUS)
-UTERUS-
1. VEHIKEL
2. VERBINDUNG A
3. VERBINDUNG B
4. ESTRADIOL
mg / 10 g KÖRPERGEW.
50
40
30
20
10
0
1
2
3
4

Beispiel 1)

11-(14$\alpha$,17$\alpha$-Ethano-3,17-dihydroxy-1,3,5,(10)-estratrien-7$\alpha$-yl)-undecansäure-(N-n-butyl-N-metyl)-amid.

a) 11-Bromundecyltertiärbutyldimethylsilylether.

175 g 11-Bromundecanol (Fluka) werden in 420 ml Tetrahydrofuran gelöst und bei 25° mit 101 g Imidazol sowie 130,1 g Tertiärbutyldimethylsilylchlorid in 140 ml Tetrahydrofuran versetzt und 2 h bei 25° gerührt. Zur Aufarbeitung gibt man 1,2 l Diethylether hinzu, filtriert das ausgefallene Hydrochlorid ab, engt im Vakuum zur Trockne ein und chromatographiert an Kieselgel mit Hexan/Essigester. Man erhält 509,6 g 11-Bromundecyltertiärbutyldimethylsilylether als Öl.

b) 7-(11-Dimethyltertiärbutylsilyloxy)-undecyl)-4-estren-3,17-dion.

7,4 g Magnesiumspäne werden in 31 ml Tetrahydrofuran vorgelegt und mit einer Lösung von 113 g 11-Bromundecyltertiärbutyldimethylsilylether in 310 ml Tetrahydrofuran innerhalb von 1,5 h versetzt. Nach 1 h bei 100° Badtemperatur wird mit 157 ml Tetrahydrofuran verdünnt, auf -30° abgekühlt, mit 29,5 g Kupfer-(I)iodid versetzt, 0,2 h bei -30° gerührt, eine Lösung von 25 g 4,6-Estradien-3,17-dion (Kalvoda, J. und Anner, G. *Helv. Chim. Acta* 50, (1967), S. 269) in 220 ml Tetrahydrofuran zugetropft und 1,5 h bei -30° weitergerührt. Dann werden 18,7 ml Eisessig zugesetzt, im Vakuum eingeengt, auf Wasser gegeben, über Celite abgesaugt, mit Essigester nachgewaschen, zweimal mit Essigester extrahiert, mit Wasser und Kochsalzlösung gewaschen, über Natriumsulfat getrocknet, im Vakuum zur Trockne eingeengt und an Kieselgel mit Hexan/Essigester chromatographiert. Man erhält 88,6 g 7$\alpha$-(11-(Dimethyltertiärbutylsilyloxy)-undecyl)-4-estren-3,17-dion $[\alpha]_D$ = 54,4° als Öl sowie 55,2 g 7$\beta$-(11-(Dimethyltertiärbutylsilyloxy)-undecyl)-4-estren-3,17-dion vom Schmelzpunkt 65°.

c) 7$\alpha$-(11-Acetoxyundecyl)-4-estren-3,17-dion.

28,3 g 7$\alpha$-(11-(Dimethyltertiärbutylsilyloxy)-undecyl)-4-estren-3,17-dion werden in 141 ml Tetrahydrofuran mit 156 ml Eisessig und 78 ml Wasser 2 h bei 50° gerührt. Dann wird im Vakuum zur Trockne eingeengt. Das auf diese Weise erhaltene rohe 7$\alpha$-(11-Hydroxyundecyl)-4-estren-3,17-dion wird mit 140 ml Pyridin und 70 ml Essigsäureanhydrid 15 h bei 25° gerührt. Zur Aufarbeitung wird auf 0° gekühlt, mit 26 ml Wasser versetzt, 0,7 h bei 0° gerührt, mit Diethylether verdünnt, mit Natriumhydrogencarbonat und Kochsalzlösung gewaschen, über Natriumsulfat getrocknet, im Vakuum zur Trockne eingeengt und an Kieselgel mit Dichlormethan/Essigester chromatographiert. Man erhält 22,4 g 7$\alpha$-(11-Acetoxyundecyl)-4-estren-3,17-dion $[\alpha]_D$ = 62,4° als Öl.

d) 3-Acetoxy-7$\alpha$-(11-acetoxyundecyl)-estra-1,3,5(10)-trien-17-on.

10,5 g 7$\alpha$-(11-Acetoxyundecyl)-4-estren-3,17-dion in 58,5 ml Acetonitril werden zu einer Lösung von 9,1 g Kupfer(II)bromid und 1.78 g Lithiumbromid in 141 ml Acetonitril gegeben und 0,5 h am Rückfluß erhitzt. Dann wird auf 0° gekühlt, langsam mit 235 ml Natriumhydrogencarbonatlösung versetzt, dreimal mit Essigester extrahiert, mit Wasser und Kochsalzlösung gewaschen, über Natriumsulfat getrocknet, im Vakuum zur Trockne eingeengt und an Kieselgel mit Hexan/Essigester chromatographiert. Man erhält 7.5 g 3-Acetoxy-7$\alpha$-(11-acetoxyundecyl)-1,3,5(10)-estratrien-17-on $[\alpha]_D$ = 75.8° als Öl.

e) 3-Acetoxy-7$\alpha$-(11-acetoxyundecyl)-17,17-ethylendioxy-1,3,5(10)-estratrien.

7.85 g 3-Acetoxy-7$\alpha$-(11-acetoxyundecyl)-1,3,5(10)-estratrien-17-on werden in 51 ml Dichlormethan und

51 ml Ethylenglycol mit 24 ml Trimethylorthoformiat und 159 mg para-Toluolsulfonsäure 0,5 h am Rückfluß gerührt. Zur Aufarbeitung wird mit Dichlormethan verdünnt, mit Natriumhydrogencarbonat- und Kochsalzlösung gewaschen, über Natriumsulfat getrocknet, im Vakuum zur Trockne eingeengt und am Kieselgel mit Hexan/Essigester chromatographiert. Man erhält 6,5 g reines 3-Acetoxy-7α-(11-acetoxyundecyl)-17,17-ethylendioxy-1,3,5(10)-estratrien $[\alpha]_D$ = 13,4° als Öl.

f) 3-Acetoxy-7α-(11-acetoxyundecyl-16α-brom-17,17-ethylendioxy-1,3,5(10)-estratrien.

6,4 g 3-Acetoxy-7α-(11-acetoxyundecyl)-17,17-ethylendioxy-1,3,5(10)-estratrien werden in 116 ml Tetrahydrofuran bei 0° mit 9,31 g Pyridinhydrobromidperbromid portionsweise versetzt. Dann wird mit Essigester verdünnt, mit Natriumsulfitlösung, Wasser und Kochsalzlösung gewaschen, über Natriumsulfat getrocknet, im Vakuum zur Trockne eingeengt und an Kieselgel mit Hexan/Essigester chromatographiert. Man erhält 5,32 g reines 3-Acetoxy-7α-(11-acetoxyundecyl)-16-brom-17,17-ethylendioxy-1,3,5(10)-estratrien $[\alpha]_D$ = 18,8° als Öl.

g) 7α-(11-Hydroxyundecyl)-17,17-ethylendioxy-1,3,5(10),15-estratetraen-3-ol.

5,32 g 3-Acetoxy-7α-(11-acetoxyundecyl)-16β-brom-17,17-ethylendioxy-1,3,5(10)-estratrien werden in 80 ml Dimethylsulfoxid und 8 ml Methanol mit 8 g Kaliumhydroxid, 6,5 h bei 90° Badtemperatur gerührt. Dann wird mit Eis/Kochsalzwasser gefällt, in Dichlormethan aufgenommen, neutralgewaschen, über Natriumsulfat getrocknet, im Vakuum zur Trockne eingeengt und an Kieselgel mit Hexan/Essigester chromatographiert. Man erhält 2,74 g reines 7α-(11-Hydroxyundecyl)-17,17-ethylendioxy-1,3,5(10),15-estratetraen-3-ol.

h) 7α-(11-Hydroxyundecyl)-1,3,5(10),15-estratetraen-3-ol-17-on.

2,6 g 7α-(11-Hydroxyundecyl)-17,17-ethylendioxy-1,3,5(10),16-estratetraen-3-ol werden in 57 ml Aceton und 6,6 ml Wasser 2,5 h mit 164 mg para-Toluolsulfonsäure bei 25° gerührt. Dann wird auf ein drittel eingeengt, mit Eis/Kochsalzwasser gefällt, abfiltriert, in Dichlormethan aufgenommen, neutralgewaschen, über Natriumsulfat getrocknet, im Vakuum zur Trockne eingeengt. Man erhält 2,3 g rohes 7α-(11-Hydroxyundecyl)-1,3,5(10),15-estratetraen-3-ol-17-on $[\alpha]_D$ = -14,0° als Öl.

j) 3,17-Diacetoxy-7α-(11-Acetoxyundecyl)-1,3,5(10),14,16-estrapentaen.

2,2 g 7α-(11-Hydroxyundecyl)-1,3,5(10),15-estratetraen-3-ol-17-on werden in 37 ml Essigsäureanhydrid mit 513 mg para-Toluolsulfonsäure 3 h bei 25° gerührt. Zur Aufarbeitung wird mit Pyridin/Kochsalzlösung gefällt, in Essigester aufgenommen, mit Natriumhydrogencarbonat- und Kochsalzlösung gewaschen, über Natriumsulfat getrocknet, im Vakuum zur Trockne eingeengt und an Kieselgel mit Hexan/Essigester chromatographiert. Man erhält 2,0 g 3,17-Diacetoxy-7α-(11-acetoxyundecyl)-1,3,5(10),14,16-estrapentaen $[\alpha]_D$ = 128,6° als Öl.

k) 3,17-Diacetoxy-7α-(11-acetoxyundecyl)-14α,17α-etheno-16β-phenylsulfonyl-1,3,5,(10)-estratrien.

1.9 g 3,17-Diacetoxy-7α-(11-acetoxyundecyl)-1,3,5(10),14,16-estrapentaen werden in 20 ml Benzol mit 1,69 g Phenylvinylsulfon 120 h bei 170° Badtemperatur im Druckbehälter gerührt. Dann wird mit Essigester in einen Glaskolben übergespühlt, am Vakuum eingengt und an Kieselgel mit Hexan/Essigester chromatographiert. Man erhält 1,61 g 3,17-Diacetoxy-7α-(11-acetoxyundecyl)- 14α,17α-etheno-16β-phenylsulfonyl-1,3,5,(10)-estratrien $[\alpha]_D$ = 76,6° als Schaum.

l) 14α,17α-Etheno-7α-(11-hydroxyundecyl)-1,3,5,(10)-estratrien-3,17-diol.

1,3 g 3,17-Diacetoxy-7α-(11-acetoxyundecyl)-14α,17α-etheno-16β-penylsulfonyl-1,3,5,(10)-estratrien in Tetrahydrofuran werden bei -78° in 50 ml flüssigen Ammoniak getropft, mit 290 mg Lithium portionsweise

versetzt und 2 h bei -70° gerührt. Dann wird mit 15 ml Ammoniumchloridlösung bei -50° versetzt, der Ammoniak abgedampft, mit Essigester verdünnt, mit Wasser und Kochsalzlösung gewaschen, über Natriumsulfat getrocknet, im Vakuum zur Trockne eingeengt und an Kieselgel mit Hexan/Essigester chromatographiert. Man erhält 0,452 g 14α,17α-Etheno-7-(11-hydroxyundecyl)-1,3,5,(10)-estratrien-3,17-diol $[\alpha]_D$ = 86,3° als Öl.

m) 14α,17α-Ethano-7α-(11-hydroxyundecyl)-1,3,5,(10)-estratrien-3,17-diol.

520 mg 14α,17α-Etheno-7α-(11-hydroxyundecyl)-1,3,5,(10)-estratrien-3,17-diol werden in 10 ml Essigester gelöst und mit 100 mg Palladium 10% auf Kohle 2,5 h bei 25° und 1 bar hydriert. Zur Aufarbeitung wird über Cellite abgesaugt mit Essigester nachgewaschen, über Natriumsulfat getrocknet, im Vakuum zur Trockne eingeengt und an Kieselgel mit Hexan/Essigester chromatographiert. Man erhält 400 mg reines 14α,17α-Ethano-7α-(11-hydroxyundecyl)-1,3,5,(10)-estratrien-3,17-diol als Schaum.

n) 3-Benzoyloxy-14α,17α-ethano-7α-(11-hydroxyundecyl)-1,3,5,(10)-estratrien-17-ol.

1,0 g 14α,17α-Ethano-7α-(11-hydroxyundecyl)-1,3,5,(10)-estratrien-3,17-diol werden in 11,3 ml Aceton bei 0° mit 3,2 ml 0,1 n-Natronlauge und 0,3 ml Benzoylchlorid versetzt und 0,5 h bei 0° nachgerührt. Zur Aufarbeitung wird auf Natriumhydrogencarbonat gegeben, dreimal mit Essigester extrahiert, mit Kochsalzlösung gewaschen, über Natriumsulfat getrocknet, im Vakuum zur Trockne eingeengt und an Kieselgel mit Hexan/Essigester chromatographiert. Man erhält 1,0 g 3-Benzoyloxy-14α,17α-ethano-7α-(11-hydroxyundecyl)-1,3,5,(10)-estratrien-17-ol $[\alpha]_D$ = 42,1° als Schaum.

o) 11-(3-Benzoyloxy-14α,17α-ethano-17-hydroxy-1,3,5,(10)-estratrien-7α -yl)-un decansäure.

1,0 g 3-Benzoyloxy-14α,17α-ethano-7α-(11-hydroxyundecyl)-1,3,5,(10)-estratrien-17-ol werden in 17 ml Aceton bei 0° langsam mit 1,0 ml Jones-Reagens versetzt und 0,5 h gerührt. Zur Aufarbeitung werden 1,3 ml 2-Propanol zugegeben, im Vakuum zur Trockne eingeengt, 0,1 n Salzsäure zugegeben, viermal mit Dichlormethan extrahiert, mit Kochsalzlösung gewaschen, über Natriumsulfat getrocknet, im Vakuum zur Trockne eingeengt und an Kieselgel mit Dichlormethan/Aceton chromatographiert. Man erhält 860 mg 11-(3-Benzoyloxy-14α,17α-ethano-17-hydroxy-1,3,5,(10)-estratrien-7α-yl-undecansäure $[\alpha]_D$ = 39,8° als Schaum.

p) 11-(3-Benzoyloxy-14α,17α-ethano-17-hydroxy-1,3,5,(10)-estratrien-7α-yl)-undecansäure-(N-n-butyl-N-metyl)-amid.

810 mg 11-(3-Benzoyloxy-14α,17α-ethano-17-hydroxy-1,3,5,(10)-estratrien-7α-yl)-undecansäure werden in 14 ml Dichlormethan bei -10° mit 0,21 ml N-Methylmorpholin und 0,24 ml Chlorameisensäureisobutylester 0,5 h gerührt. Anschließend werden 0,27 ml N-Methylbutylamin langsam zugetropft und 1 h bei 25° gerührt. Zur Aufarbeitung wird mit Natriumhydrogencarbonatlösung versetzt, mit Dichlormethan verdünnt, mit Wasser und Kochsalzlösung gewaschen, über Natriumsulfat getrocknet, im Vakuum zur Trockne eingeengt und an Kieselgel mit Hexan/Essigester chromatographiert. Man erhält 631 mg 11-(3-Benzoyloxy-14α,17α-ethano-17-hydroxy-1,3,5,(10)-estratrien-7α-yl)-undecansäure-(N-n-butyl-N-metyl)-amid.

q) 11-(14α,17α-ethano-3,17-dihydroxy-1,3,5,(10)-estratrien-7α-yl)- undecansäure-(N-n-butyl-N-metyl)-amid.

601 mg 11-(3-Benzoyloxy-14α,17α-ethano-17-hydroxy-1,3,5,(10)-estratrien-7α-yl)-undecansäure-(N-n-butyl-N-metyl)-amid werden in 10 ml Methanol und 5 ml Tetrahydrofuran mit 5 ml 1n Natronlauge 0,5 h bei 25° gerührt. Dann wird mit 1n-Salzsäure neutralisiert, im Vacuum eingeengt, auf Wasser gegeben, viermal mit Dichlormethan extrahiert, mit Kochsalzlösung gewaschen, über Natriumsulfat getrocknet, im Vakuum zur Trockne eingeengt und an Kieselgel mit Dichlormethan/Aceton chromatographiert. Man erhält 593 mg 11-(14α,17α-ethano-3,17-dihydroxy-1,3,5,(10)-estratrien-7α-yl)-undecansäure (N-n-butyl-N-metyl)-amid $[\alpha]_D$ = 22,7° als Schaum.

## Ansprüche

1. 14, 17α-Etheno- und -Ethanoestratriene der allgemeinen Formel I

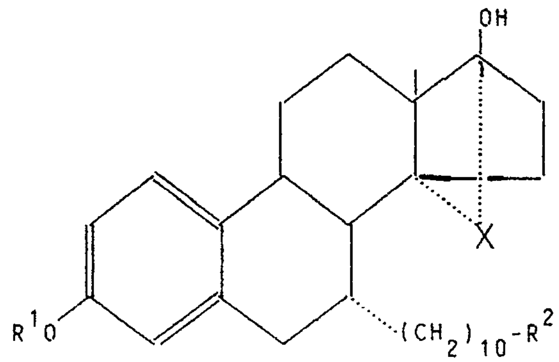

(I),

worin
X für eine $-CH_2-CH_2-$ oder

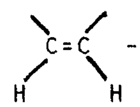

Brücke,
$R^1$ für ein Wasserstoffatom, eine $C_1$- bis $C_{15}$-Acyl-, Benzoyl- , $C_1$- bis $C_{15}$-Alkyl-, $C_3$- bis $C_9$-Cycloalkyl- oder Alkylcycloalkylgruppe und
$R^2$ für die Gruppierung

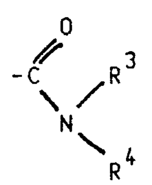

oder

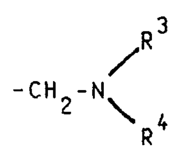

,

wobei $R^3$ und $R^4$ unabhängig voneinander ein Wasserstoffatom oder eine geradkettige oder verzweigte, gegebenenfalls teilweise oder vollständig fluorierte $C_1$-$C_8$-Alkylgruppe bedeuten,
stehen.

2. 14,17α-Etheno- und -Ethanoestratriene nach Anspruch 1, worin $R^1$ für ein Wasserstoffatom, eine $C_1$- bis $C_6$-Acyl-, Benzoyl-, $C_1$- bis $C_6$-Alkyl- oder $C_3$-bis $C_6$-Cycloalkylgruppe steht.

3. 14,17α-Etheno- und -Ethanoestratriene nach Anspruch 1, worin $R^1$ für ein Wasserstoff, eine Acetyl- oder Methylgruppe steht.

4. 14,17α-Etheno- und -Ethanoestratriene nach Anspruch 1, worin $R^3$ für eine $C_1$-bis $C_8$-Alkyl- und $R^4$ für eine teilweise oder vollständig fluorierte $C_1$- bis $C_8$-Alkylgruppe stehen.

5. 14,17α-Etheno- und -Ethanoestratriene nach Anspruch 4, worin $R^3$ für eine Methyl- uznd $R^4$ für eine 2,3,4-Heptafluorbutylgruppe stehen.

6. 11-(14α,17α-Ethano-3,17-dihydroxy-1,3,5(10)-estratrien-7α-yl)-undecansäure-(N-n-butyl-N-methyl)-amid.
11-(14α, 17α-Ethano-3,17-dihydroxy-1,3,5(10)-estratrien-7α-yl)-undecansäure-(N-2,3,4-heptafluor-n-butyl-N-methyl)-amid.

7. Verfahren zur Herstellung von Verbindungen der allgemeinen Formel I

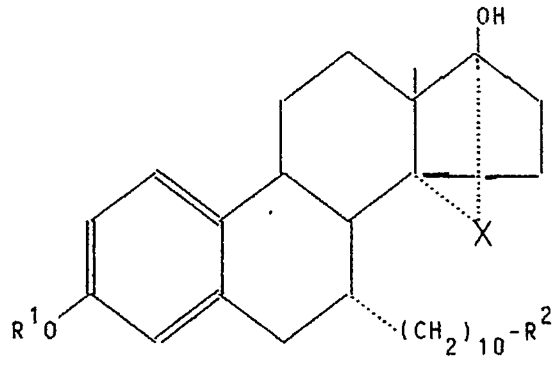

(I),

worin X, $R^1$ sowie $R^2$ die in Anspruch 1 angegebene Bedeutung haben, dadurch gekennzeichnet, daß in der Verbindung der allgemeinen Formel II

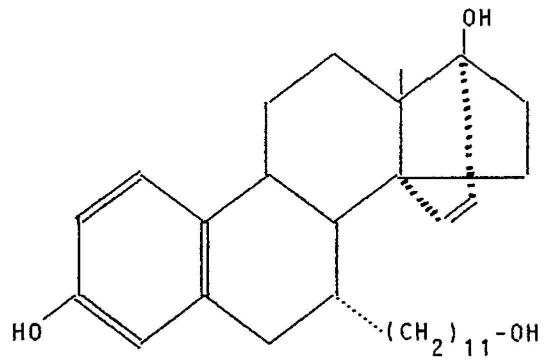

(II),

die Doppelbindung der 14α,17α-Ethenobrücke gegebenenfalls hydriert, die 3-Hydroxyfunktion geschützt, die 11-Hydroxygruppe der 7α-Undecylseitenkette zur Carbonsäure oxidiert, mit dem Amin $H\text{-}NR^3R^4$, worin $R^3$ und $R^4$ die in Formel I bei der Definition der Gruppierung $R^2$ angegebene Bedeutung haben, aus der Carbonsäure das entsprechende Carbonsäureamid hergestellt, die Schutzgruppe in 3-Stellung gegebenenfalls abgespalten und anschließend gegebenenfalls die 3-Hydroxygruppe entweder partiell acetyliert oder methyliert und/oder gegebenenfalls die Ketogruppe des Carbonsäureamids vollständig reduziert wird.

8. Pharmazeutische Präparate, dadurch gekennzeichnet, daß sie mindestens eine Verbindung gemäß Anspruch 1 bis 6 sowie einen pharmazeutisch verträglichen Träger enthalten.

9. Verwendung mindestens einer Verbindung gemäß Anspruch 1 bis 6 zur Herstellung von Arzneimitteln zur Behandlung von estrogen-bedingten oder -induzierten Krankheiten.

10. Verwendung mindestens einer Verbindung gemäß Anspruch 1 bis 6 zur Herstellung von Arzneimitteln zur Behandlung der anovulatorischen Infertilität.

11.Verwendung mindestens einer Verbindung gemäß Anspruch 1 bis 6 zur Herstellung von Arzneimitteln zur Prophylaxe und Therapie der Prostatahyperplasie.

12.Verwendung mindestens einer Verbindung gemäß Anspruch 1 bis 6 zur Herstellung von Arzneimitteln zur Behandlung des Mammacarcinoms, des Endometriumcarcinoms und von Melanomen.